# EUROPEAN PATENT APPLICATION

(11) **EP 2 574 658 A1**
(43) Date of publication of application: **03.04.2013**
(21) Application number: 11183352.1
(22) Date of filing: 29.09.2011
(51) Int. Cl.: C12M 1/00

(54) **Arrangement for handling a plurality of containers**

(71) Applicant: Rheinisch-Westfälische Technische Hochschule Aachen (RWTH), 52062 Aachen (DE); Myrenne GmbH, 52159 Roetgen (DE)
(72) Inventor: Jockenhövel, Stefan, 52072 Aachen (DE); van Gilse, Jan, 52076 Aachen (DE); Schmitz-Rode, Thomas, 52070 Aachen (DE)
(74) Representative: Remus, Alvaro Johannes

(57) **Abstract**

The invention relates to an arrangement 20 for handling a plurality of containers, in particular cell culture flasks and/or multiwell plates, that are arranged on at least one rack within an inner space 22 of an incubator 21, the arrangement 20 comprising at least one element for interacting with the rack and at least one member for manipulating at least one of the containers. According to the invention, the arrangement 20 is a mobile unit that is compatible with a plurality of different types of incubators 21.

## Description

### Background of the invention

The invention relates to an arrangement for handling a plurality of containers, in particular cell culture flasks and/or multiwell plates, that are arranged on at least one rack within an inner space of an incubator, wherein the arrangement comprises at least one element for interacting with the rack and at least one member for manipulating at least one of the containers. The invention further concerns a rack that is compatible with the arrangement and a method for handling a plurality of containers.

### Prior art

Culturing of cells, in particular animal and human cells, is a time-consuming process that requires human resources to a great extend. Typically, cells are cultivated by supplying a liquid culture medium including nutrients and essential growth factors into a culture container, for example, a culture flask or multiwell plate. The cells are inoculated into this liquid medium and then cultivated in an incubator under appropriate conditions. As the cells consume the nutrients and release metabolites into the medium, an exchange of medium, wherein the used culture medium is discarded and replaced by fresh medium, is necessary periodically. Further, especially in cultivation of attachment-dependent cells, the cells adhere to the bottom of the container and cell growth is inhibited when confluence is reached, i.e. when most of the cells get in contact to their respective neighbor(s). Accordingly, cell passaging is needed to avoid contact inhibition. To this end, the cells are detached from the container bottom by enzymatic treatment, diluted and subsequently distributed into several new containers. Finally, if the desired total amount of cells is reached, the cells are harvested and supplied for their intended purpose.

In any case, all this work is mostly accomplished manually by highly trained personnel so that time and effort as well as costs are very high. Additionally, manually handling of cell culture containers always implies the risk of contamination. Therefore, stand-alone systems including robots for handling cell culture containers have been developed.

EP-A-1 661 980 discloses an automatic cell cultivation apparatus which is capable of conveying culture containers, exchanging the culture medium, and operating other jobs promptly by a robot. This stand-alone apparatus includes a main body for cultivating cells in a culture container which is equipped with one or more incubators accommodating the culture containers, a chemical suction machine for sucking chemicals into the culture container, and a multijoint robot for conveying the culture container. The multijoint robot has functions of conveying a culture container, detaching and attaching the container's lid, and inclining the container when sucking chemicals into it.

JP-A-2009 291104 discloses another automatic cell culture apparatus operated by an operation robot. The apparatus represents a stand-alone system including an incubator, a refrigeration storage, a capper, an operation station, a temporary station, a chip stand, and a centrifugal separator. These components are arranged along an installation surface in parallel with the backside of a case body which is disassembled into three units. A rail is arranged along an installation surface and a lifting and lowering post is arranged perpendicular to this rail. The lifting and lowering post is equipped with the operation robot which is movable along the installation surface in horizontal and vertical direction.

Further, JP-A-2002 262856 discloses another stand-alone system, with which culture containers that are arranged on racks within an incubator are taken out of this rack by an orthogonal carrier robot and then conveyed to a predetermined position. This system for automatically exchanging culture medium further includes a culture medium-exchanging robot equipped with a culture medium-injecting needle and a culture medium-discharging needle. In particular, the culture containers are removed from the incubator by the carrier robot to discharge the liquid culture medium from the container using the medium-discharging needle and to inject fresh liquid culture medium into the container with the medium-injecting needle of the medium-exchanging robot. Finally, the processed containers are conveyed to the rack by the carrier robot and placed there again for further incubation.

However, all these stand-alone systems are very expensive and bulky devices. It is a further drawback of the prior art systems that they cannot be easily integrated into existing laboratory facilities or used with common incubators. Therefore, existing laboratory equipment, in particular incubators, have to be replaced with the known automated culture container handling systems and cannot be used furthermore. This renders the prior art systems economically inefficient and thus unattractive especially for smaller companies and non-profit research organizations.

### Summary of the invention

It is an object of the invention to provide an arrangement for handling a plurality of containers which can be easily integrated into existing laboratory facilities and used with common incubators.

This object is achieved by an arrangement of the initially-mentioned kind which is a mobile unit that is compatible with a plurality of different types of incubators. As the arrangement according to the invention is a mobile unit, it can be easily integrated into existing laboratory facilities and moved to any place in the laboratory. In particular, the arrangement according to the invention can be moved to different incubators so that the capacity of the laboratory may be easily expanded with low costs by acquiring additional incubators. Due to the fact that the arrangement according to the invention is devoid of an incubator, it has a compact design so that undesired remodeling of the existing space and facilities can be avoided. Being compatible with a plurality of different types of incubators is also advantageous because the arrangement according to the invention can be used with almost any common incubator. Accordingly, existing laboratory equipment, in particular incubators, can be maintained and there is no need to replace them with a completely new system. Even if different types of incubators are used by different groups within a company or organization, only one or a few arrangements according to the invention are necessary to satisfy all groups since the arrangement can be moved to and used with the different incubators. Consequently, the overall cost incurred with the purchase of automated cell culture systems is dramatically reduced so that the arrangement according to the invention is very useful for smaller companies and non-profit research organizations such as universities.

In a preferred embodiment of the invention, the arrangement according to the invention further comprises at least one coupling member for establishing a connection to the incubator, preferably to a corresponding coupling part of the incubator. This coupling member may comprise, for example, a magnetic or magnetisable element, which ensures a close and stable connection of the arrangement to the incubator, and/or a latch lock for establishing a mechanical connection to the incubator. Additionally or alternatively, at least one frame-like element for adapting an opening of the arrangement to the dimensions of an opening of the incubator is provided. As different incubators often have differently sized openings, it is necessary to adapt the opening of the arrangement to the dimensions of the incubator. This can be advantageously achieved by a frame-like element surrounding at least one opening of the arrangement, which has a width that is sufficient to compensate the difference in size between the openings of the incubator and the arrangement. Accordingly, close and airtight connection of the arrangement according to the invention to differently sized incubators can be ensured. Preferably, the frame-like element comprises a washer, for example a rubber washer. In a preferred embodiment, the coupling member is integrated into the frame-like element of the arrangement.

In another preferred embodiment of the invention, the arrangement according to the invention further comprises at least one elevating means for adjusting the height of the arrangement. The elevating means advantageously ensures easy adaption of the arrangement to the height and/or position of the existing incubator. The elevating means preferably comprises a hydraulic elevating device which may be operated by an electric motor. Alternatively, the elevating means may comprise an electromechanical drive or a mechanical mechanism that is operated manually.

In a further preferred embodiment of the invention, the arrangement according to the invention further comprises at least one separating element for separating an inner space of the arrangement from the inner space of the incubator. Usually, the air within the inner space of an incubator has a controlled temperature and gas composition adapted to the special needs of the cultured cells, e.g., 37 °C and 5 % CO₂. However, as the containers are usually sealed by a sterile filter, it is not necessary to keep the incubator sterile. In contrast, it is preferred to keep the air within the inner space of the arrangement according to the invention sterile, for example, by a laminar flow system, but it is not necessary to control its temperature or composition. Thus, the different atmospheres have to be separated from each other when the arrangement is coupled to an incubator. This can be achieved by a separating element which ensures airtight sealing, preferably a separating wall made of a suitable polymer and/or glass. In order to allow a transfer of containers from the incubator into the arrangement, a window or the like in the separating element may be provided, preferably a sliding window.

In order to render at least one container accessible for processing, it is preferred that the interacting element comprises means that move the rack and/or at least one of the containers into an appropriate position. For instance, this means may comprise a rotary drive and a crank, which turn the rack until a desired row of containers is accessible.

The arrangement according to the invention may further comprise a grappler or conveyer capable of removing at least one of the containers from the rack. Using a grappler, a conveyer or the like, the containers can be easily removed from the rack and transferred from the incubator to a manipulating member of the arrangement for further processing.

Preferably, the manipulating member comprises a combined decapping/capping unit so that the containers can be opened for filling in and/or removing of medium and/or cells and finally closed after processing for (further) incubation.

In a preferred embodiment of the invention, the manipulating member comprises a pour and/or filling unit, preferably a swivel device, which is/are capable of removing and/or filling medium and/or cells from/into the container. The pour unit may comprise a retainer for holding at least one container and a mechanism for pivoting the container. Pivoting the container is a preferred option to pour liquid, e.g. used medium, out of the container. If discharged, the container may be filled again with fresh medium by a filling unit which may comprise a pumping device and a nozzle for introducing a liquid into the container. Preferably, the filling unit comprises a pour unit which includes a retainer for holding a reservoir and a mechanism for pivoting the reservoir. In this embodiment, the liquid, in particular the fresh medium, is automatically poured from the reservoir into the container until the desired filling level of the container is reached. Obviously, the pour and/or filling unit of the manipulating device can also be used for cell passaging and harvesting accordingly.

If liquid is filled and/or poured into the container, the filling level may be measured by at least one optical sensor. The filling unit may be controlled such that the filling process is stopped when the preset filling level is reached. However, in a preferred embodiment of the invention, the arrangement according to the invention further comprises at least one weighing device for measuring the weight of the container (including liquid), preferably for determining the filling level within the container. In this embodiment, the container is placed on the weighting device and the liquid is filled into the container until the weighting device measures a preset value that is a little bit lower, for example 10 % lower, than the desired weight (preset total weight of the container with liquid) representing the actual volume of the liquid in the container (total weight of the container with liquid minus weight of the container alone, converted into the volume of the liquid). After this initial step of rough filling, small volumes of the liquid are stepwise filled into the container until the desired weight is reached. Accordingly, precise filling of the container can be achieved.

Moreover, if liquid (e.g. a cell suspension) has to be transferred from a reservoir or source container to one or more target container(s), e.g. during passaging of cells or whenever the content of a container (e.g. cell culture flask) has to be equally, i.e. in aliquots, distributed into at least two target containers (e.g. cell culture flasks), this is advantageously accomplished by means of a swivel device which holds at least one source container and is designed to pivot this source container around a swivel axis. By pivoting the source container, a predefined volume of the liquid is poured into a first target container, wherein the predefined volume is controlled by the weighting device as described above. Subsequently, the first target container is replaced by a second target container which is also filled with the predefined volume of liquid. This process continues until the liquid is completely distributed from the source container(s) to all target containers. In this method, while pivoting the source container, the point of time when the liquid in the source container reaches the rim of the container is detected by a sensor device so as to control the pouring process accurately. In a preferred embodiment of the invention, this sensor device is a capacitive sensor. The sensor device may be disposed on a movable holding member so that the sensor can be moved to a detecting position near the rim of the container before the pouring process begins and back to a waiting position if the sensor would otherwise form an obstacle. In any case, if compared to commonly used pipetting systems for changing liquids or passaging cells, the swivel device and pouring method according to the invention minimize the risk of cross-contamination and significantly reduce the consumption of disposables, e.g. pipettes. Thus, operational costs of the system are permanently reduced.

In a another preferred embodiment of the invention, the arrangement according to the invention further comprises at least one sterile filter element and a laminar flow system. By these components, the air within at least one inner space of the arrangement can be kept sterile so as to avoid contamination of the cell cultures during handling of the containers. In this embodiment, a slight overload pressure may be generated within the inner space by means of a pressure-controlled fan so as to avoid undesired entry of (contaminated) air from the surrounding atmosphere through necessary openings. As a result, inoculation of cells, change of medium, cell passaging, and cell harvesting can be accomplished within the arrangement according to the invention under aseptic condition.

In a further preferred embodiment of the invention, the arrangement according to the invention further comprises a cooled reservoir for liquids and a heating element for heating liquids. Accordingly, cell culture media can be stored over a longer period of time and heated up to a desired temperature, e.g. 37 °C, shortly before they are used.

One aspect of the invention concerns a rack which is compatible with the arrangement according to the invention and capable of holding at least one container. This rack comprises at least one member corresponding to at least one interacting element of the arrangement so that it is perfectly adapted to the arrangement. For example, the rack may comprise a coupling in which a crank driven by a rotary drive can engage so as to turn the rack into an appropriate position.

In a preferred embodiment, the rack further comprises at least one means for reversibly mounting it to the inner walls of an incubator. Additionally, the rack is adapted or at least adaptable to the dimensions of the inner space of the incubator. Accordingly, the rack can be easily mounted within different types of incubators.

Another aspect of the invention concerns a method for handling a plurality of containers, in particular cell culture flasks and/or multiwall plates. This method comprises:
a) Moving the arrangement according to the invention to a first incubator,
b) connecting the arrangement to the first incubator, and
c) automatically handling at least one container.
In a preferred embodiment of the method according to the invention, the method further comprises:
d) Disconnecting the arrangement from the first incubator,
e) moving the arrangement to a second incubator,
f) connecting the arrangement to the second incubator, and
g) automatically handling at least one container, and
h) optionally connecting the arrangement subsequently to a plurality of incubators, particularly incubators of different type and/or size.

The invention is further exemplarily described in detail with reference to the figures.

### Brief description of the figures

Figure 1 shows a schematic side view of an arrangement according to the invention connected to a common incubator (side wall of the incubator omitted).
Figure 2 shows a perspective view of an arrangement according to the invention and a common incubator.

### Description of exemplary and preferred embodiments of the invention

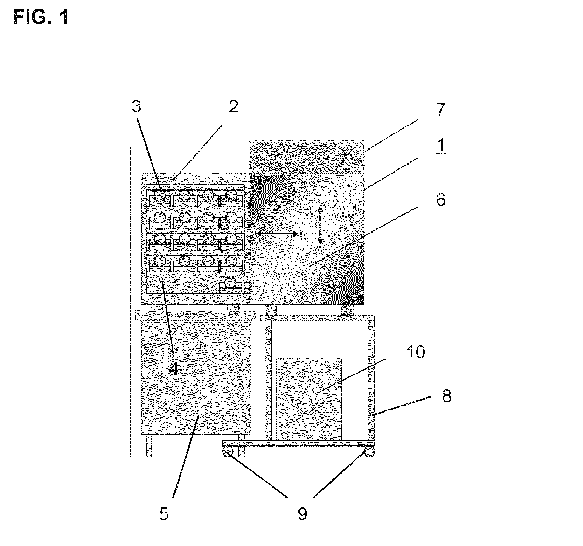

**Figure 1** shows an exemplary embodiment of an arrangement 1 according to the invention which is connected to an incubator 2. The incubator 2 is a common incubator as usually used in cell culture laboratories. Cell culture flasks 3 are stacked within the inner space 4 of the incubator 2, preferably arranged on a suitable rack (not shown). The incubator 2 is placed on a board 5.

The arrangement 1 according to the invention has an inner space 6 where cell culture flasks 3 removed from the incubator 2 are handled. A laminar flow system including at least one sterile filter element 7 ensures that the flasks 3 can be handled under aseptic condition. The arrows indicate that the arrangement 1 can be moved in horizontal direction and elevated vertically. To this end, the arrangement 1 is arranged on a support frame 8 which comprises wheels 9 and elevating means (not shown). The elevating means preferably comprises a hydraulic elevating device which may be operated by an electric motor. By means of these position-adjusting components the arrangement 1 can be easily positioned such that its opening for loading the flasks 3 substantially fits to the opening of the incubator 2, irrespective of the type of the incubator 2 or its dimensions and position. The support frame 8 also serves as a support for a refrigerator 10 which can be used as storage, for example, for liquid cell culture medium.

As the arrangement 1 for automatically handling cell culture containers is a mobile unit, it can be easily integrated into existing laboratory facilities and moved to any place in the laboratory. Moreover, the arrangement 1 is adjustable by wheels 9 and elevating means (not shown) so that is compatible with a plurality of different types of incubators independent of their size and position. Thus, the arrangement 1 can be used with existing laboratory equipment, in particular ordinary incubators, so that there is no need to replace the existing equipment with an expensive and bulky stand-alone system for handling cell cultures.

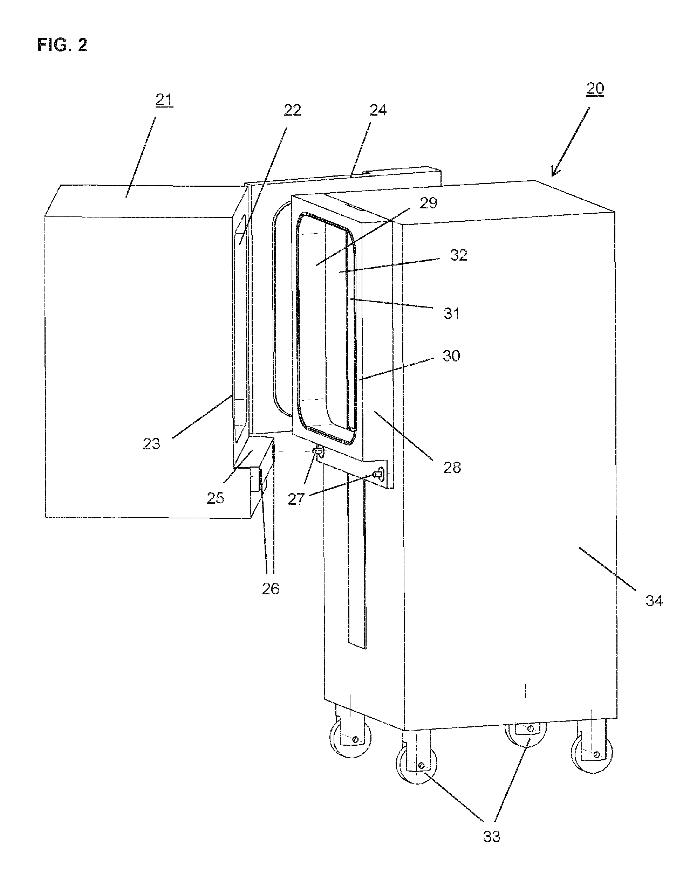

**Figure 2** shows another exemplary embodiment of an arrangement 20 according to the invention which is positioned near to a common incubator 21. The incubator 21 is a common incubator as usually used in cell culture laboratories. Cell culture flasks (not visible) are arranged on a rack (not visible) within the inner space 22 of the incubator 21. The incubator 21 may be arranged on a laboratory bench (not shown) or any other suitable support. For coupling the arrangement 20 to the opening 23 of the incubator 21, the door 24 of the incubator 21 is opened with an angle of at least 90°. The incubator 21 further comprises a bar-like protrusion 25 underneath the opening 23, which comprises two cavities 26 corresponding to respective coupling members of the arrangement 20. Preferably, the bar-like protrusion 25 is a separate coupling part which can be mounted on every common type of incubator in order to render the respective incubator compatible with the arrangement according to the invention.

As coupling members, the arrangement 20 comprises two bolt-like pins 27 which fit into the cavities 26 of the bar-like protrusion 25 of the incubator 21. In the embodiment according to figure 2, the bolt-like pins 27 are integrated in a frame-like element 28 of the arrangement 20. This frame-like element 28 adapts the opening 29 of the arrangement 20 to the dimensions of the opening 23 of the incubator 21. Usually, different types of incubators have differently sized openings so that it is necessary to adapt the opening of the arrangement according to the invention to the dimensions of the incubator, to which it shall be connected. To this end, the frame-like element 28 surrounding the opening 29 of the arrangement 20 has a width that is sufficient to compensate the differences between different types of incubators. Preferably, the frame-like element 28 and/or the bolt-like pins 27 comprise at least one magnetic or magnetisable element. By means of the attractive force of this (these) magnetic or magnetisable element(s), the arrangement 20 can be attached to the incubator 21 so that a close and stable connection of the arrangement 20 to the incubator 21 can be ensured. Alternatively, a latch lock may be provided, which establishes a mechanical connection of the arrangement 20 to the incubator 21. In order to ensure a close and airtight connection of the arrangement 20 to the incubator 21, the frame-like element 28 comprises a washer 30, for example a rubber washer.

The arrangement 20 further comprises at least one separating element for separating the inner space 31 of the arrangement 20 from the inner space 22 of the incubator 21. In the embodiment according to figure 2, the separating element is a separating wall including two sliding doors 32 (only one door visible). In the closed state, the sliding doors 32 separate the different atmospheres within the incubator 21 (37 °C, 5 % CO₂) and the arrangement 20 (sterile) from each other when the arrangement 20 is coupled to the incubator 21. Usually, the air within the inner space of an incubator has a controlled temperature and gas composition adapted to the special needs of the cultured cells, while the air within the inner space of an arrangement according to the invention has to be sterile as cell culture containers and/or medium reservoirs have to be opened during processing of the cell cultures. When a container is taken out of the incubator 21 and conveyed into the arrangement 20, the sliding doors 32 are opened as far as necessary to handle the container. As the air within the inner space 31 of the arrangement 20 is kept sterile by a laminar flow system, the risk of contamination when the doors 32 are open is negligible low. In order to avoid undesired entry of non-sterile air through opening 29 of arrangement 20, a slight overload pressure is generated within the inner space 31 by means of a pressure-controlled fan (not shown).

For mobility of the arrangement 20, wheels 33 are arranged at the bottom of the housing 34 of the arrangement 20. Thus, the arrangement 20 is a mobile unit that can be easily moved from one incubator to another. Additionally, for adjusting the height of the arrangement 20 and the position of the opening 29, the arrangement 20 is equipped with at least one elevating means (not shown) that is capable to move the arrangement 20 in vertical direction.

Within the inner space 31 of the arrangement 20, an interacting element (not visible) for interacting with a rack of an incubator and manipulating members (not visible) are arranged. In a preferred embodiment, the interacting element comprises means that move the rack, for example, by means of a rotary drive and a crank that engages a corresponding coupling of the rack, which turn the rack until a desired row of containers is accessible. A container to be processed is then removed from the rack by a grappler or conveyer (not shown), which is also arranged within the inner space 31 of the arrangement 20 but capable of entering the inner space 22 of the incubator 21 through the openings 29 and 23, and transferred to a manipulating member for further processing. Preferably, the manipulating member comprises a combined de-capping/capping unit so that the containers can be opened for removing used medium (or cells for passaging or harvesting) and finally closed after processing for further incubation. In this preferred embodiment, the manipulating member further comprises a pour and/or filling unit which is capable of removing used medium and filling fresh medium into the open container. The pour unit comprises a retainer for holding the container and a mechanism for pivoting the container so as to pour the used medium out of the container. If discharged, the container is filled again with fresh medium by the filling unit which comprises a pumping device and a nozzle for introducing the medium into the container. Preferably, the filling unit comprises a pour unit which includes a retainer for holding a reservoir and a mechanism for pivoting the reservoir. In this embodiment, the fresh medium is automatically poured from the reservoir into the container until the desired filling level of the container is reached. The filling level of the container is determined by a weighing device (not visible).

### List of reference numbers

- 1: Arrangement
- 2: Incubator
- 3: Cell culture flasks
- 4: Inner space
- 5: Board
- 6: Inner space
- 7: Sterile filter element
- 8: Support frame
- 9: Wheels
- 10: Refrigerator

- 20: Arrangement
- 21: Incubator 22 Inner space
- 23: Opening
- 24: Door
- 25: Protrusion
- 26: Cavities
- 27: Pins
- 28: Frame-like element
- 29: Opening
- 30: Washer 31 Inner space
- 32: Doors
- 33: Wheels
- 34: Housing

## Claims

1. Arrangement (1, 20) for handling a plurality of containers, in particular cell culture flasks (3) and/or multiwell plates, that are arranged on at least one rack within an inner space (4, 22) of an incubator (2, 21), said arrangement (1, 20) comprising at least one element for interacting with said rack and at least one member for manipulating at least one of said containers, **characterized in that** said arrangement (1, 20) is a mobile unit that is compatible with a plurality of different types of incubators (2, 21).

2. Arrangement according to claim 1, further comprising at least one coupling member for establishing a connection to the incubator, preferably to a corresponding coupling part of the incubator, and/or at least one frame-like element (28) for adapting an opening (29) of the arrangement (20) to the dimensions of an opening (23) of the incubator (21).

3. Arrangement according to claim 1 or 2, further comprising at least one elevating means for adjusting the height of the arrangement (1, 20).

4. Arrangement according to claim 1, 2 or 3, further comprising at least one separating element for separating an inner space (6, 31) of the arrangement (1, 20) from the inner space (4, 22) of the incubator (2, 21).

5. Arrangement according to any one of claims 1 to 4, wherein said interacting element comprises means that move said rack and/or at least one of said containers into a position rendering at least one container accessible.

6. Arrangement according to any one of claims 1 to 5, further comprising a grappler or conveyer capable of removing at least one of said containers from said rack.

7. Arrangement according to any one of claims 1 to 6, wherein said manipulating member comprises a combined de-capping/capping unit.

8. Arrangement according to any one of claims 1 to 7, wherein said manipulating member comprises a pour and/or filling unit, preferably a swivel device.

9. Arrangement according to any one of claims 1 to 8, further comprising at least one weighing device for measuring the weight of the container, preferably for determining the filling level within the container.

10. Arrangement according to any one of claims 1 to 9, further comprising at least one sterile filter element (7) and a laminar flow system.

11. Arrangement according to any one of claims 1 to 10, further comprising a cooled reservoir for liquids and a heating element for heating liquids.

12. Rack being compatible with the arrangement (1, 20) according to any one of claims 1 to 11 and capable of holding at least one container, said rack comprising at least one member corresponding to at least one interacting element of said arrangement (1, 20).

13. Rack according to claim 12, further comprising at least one means for reversibly mounting said rack to the inner walls of an incubator (2, 21) and being adapted or at least adaptable to the dimensions of the inner space (4, 22) of the incubator.

14. Method for handling a plurality of containers, in particular cell culture flasks (3) and/or multiwall plates, said method comprising:
a) Moving the arrangement (1, 20) according to any one of claims 1 to 9 to a first incubator (2, 21),
b) connecting the arrangement (1, 20) to said first incubator (2, 21), and
c) automatically handling at least one container.

15. Method according to claim 13, further comprising:
d) Disconnecting said arrangement (1, 20) from said first incubator (2, 21),
e) moving the arrangement (1, 20) to a second incubator,
f) connecting the arrangement (1, 20) to said second incubator, and
g) automatically handling at least one container, and
h) optionally connecting the arrangement (1, 20) subsequently to a plurality of incubators, particularly incubators of different type and/or size.
